# EUROPÄISCHE PATENTSCHRIFT

(11) **EP 2 552 413 B1**
(45) Veröffentlichungstag und Bekanntmachung des Hinweises auf die Patenterteilung: **29.04.2015**
(21) Anmeldenummer: 11717461.5
(22) Anmeldetag: 02.03.2011
(51) Int. Cl.: A61K 9/133, A61K 8/37, A61Q 19/00, A61K 8/14

(54) **NEUARTIGES TRÄGERSYSTEM FÜR DEN TRANSPORT VON WIRKSTOFFEN IN DIE HAUT**
NOVEL SUPPORT SYSTEM FOR THE TRANSPORT OF ACTIVE SUBSTANCES INTO THE SKIN
NOUVEAU SYSTÈME VECTEUR POUR LE TRANSPORT DE SUBSTANCES ACTIVES DANS LA PEAU

(30) Priorität: 26.03.2010 DE 102010013064
(43) Veröffentlichungstag der Anmeldung: 06.02.2013
(73) Patentinhaber: Blume, Gabriele, 36396 Steinau an der Strasse (DE)
(72) Erfinder: Blume, Gabriele, 36396 Steinau an der Strasse (DE)
(74) Vertreter: Böck, Bernhard
(86) Internationale Anmeldenummer: PCT/DE2011/000210
(87) Internationale Veröffentlichungsnummer: WO 2011/116738

(56) Entgegenhaltungen:
- WO-A1-00/12108
- WO-A1-96/09812
- WO-A2-03/047494
- US-A- 6 033 710
- US-A1- 2005 244 488
- "Product Information IMWITOR 375", Sasol Germany GmbH , April 2000 (2000-04), XP000002659490, Gefunden im Internet: URL:http://www.warnergraham.com/images/Imw itor375ProdInfo.pdf [gefunden am 2011-09-20]

## Beschreibung

Die vorliegende Erfindung beruht auf einer verbesserten therapeutischen Wirksamkeit gegeben durch ein neuartiges Trägersystem (Lipidvesikel), das sowohl lipophile als auch hydrophile kosmetische oder pharmazeutische Wirkstoffe beinhalten kann und diese in die tieferen Hautschichten zu transportieren vermag. Die vorliegende Erfindung betrifft auch eine das Trägersystem beinhaltende Zubereitung.

Die Art der Verkapselung von hydrophilen Wirkstoffen in Vesikeln mit einer Größe im Submikrometerbereich (nm) ist seit vielen Jahren bekannt und wird insbesondere in der Kosmetik und Pharmazie häufig eingesetzt. Vesikel, die ebenfalls die Fähigkeit besitzen, Wirkstoffe in die Haut zu transportieren, sind insbesondere die Liposomen (Phosphatidycholin / Lecithin) und Niosomen (non ionic surfactants Vesikel). Verschiedene Zusammensetzungen dieser Vesikel sind in dem Artikel "Liposomes and Niosomes as Topical Drug Delivery Systems" beschrieben (M.J. Choi & H.I. Maibach; Skin Pharmacol. Physiol. 18 (2005) 209-219). Trägersysteme, die insbesondere in der Kosmetik eingesetzt werden, führen Patravale und Mandawgade in einem Übersichtsartikel auf (Novel cosmetic delivery systems: an application update; Int. J. Cosm. Sci. 30 (2008) 19-33).

Klassische Liposomen beinhalten als membranbildende Hauptkomponente Phosphatidylcholin (PC), natürlich gewonnen aus Eiern oder Sojalecithin bzw. synthetisch hergestellt. Diese Lipide bilden spontan Vesikel (multilamellare Liposomen), wenn sie in Wasser gegeben werden. Je nach Zusammensetzung der Phospholipide (Phosphatidylcholin bestehend aus langkettigen, ungesättigten Fettsäuren) und Produktion (Hochdruckhomogenisation) können kleine einschalige und flexible Liposomen hergestellt werden. Diese Art von Liposomen ist dann befähigt, eingeschlossene Wirkstoffe in die tieferen Hautschichten zu transportieren. Artmann und andere beschreiben kleine einschalige Liposomen - hergestellt aus Sojalecithin (> 85% PC mit hohem Anteil an ungesättigten Fettsäuren, Linolsäure) - die verkapseltes hochmolekulares Heparin in die Epidermis und Dermis transportieren können (Liposomes from Soya Phospholipids as Percutaneous Drug Carriers; Arzneimittel Forschung Drug Research 40 (1990) 1365-1368). Dass der hohe Anteil an membranbildenden Phosphatidylcholin und die Flexibilität der Vesikelmembran Voraussetzungen für die Penetration der Liposomen in die Haut sind, wird deutlich im Artikel "Liposomes = Liposomes" (G. Blume; SÖFW-Journal 11 (2000) 14-17).

Eine Weiterentwicklung, um Wirkstoffe noch effizienter über die Haut in den Körper zu bringen (bis in die Gelenke), sind die "Transfersomen". Transfersomen unterscheiden sich von den herkömmlichen Liposomen durch einen Anteil an randaktiven Substanzen in der Phospholipidmembran (DE 4107153). Nach topikaler Applikation dieser ultraflexiblen Trägersysteme können die eingeschlossenen Wirkstoffe selbst aus der Dermis via lymphatischer Route ins Blut gelangen. So konnte der Zuckerspiegel im Blut nach Auftragen Insulin-haltiger Transfersomen gesenkt werden ("Transdermal Drug Carriers"; G. Cevc und andere; J. Contr. Release 36 (1995) 3-16).

Eine andere Verbesserung der Penetrationseigenschaften von kleinen flexiblen Liposomen wurde durch einen erhöhten Gehalt an Alkoholen in der Vesikelsuspension erzielt, wie in der US Patentanmeldung US 5716638 A beschrieben ist. Der hohe Anteil an Äthanol in den "Ethosomen" führt zu einer Störung / Fluidisierung der Hautlipide, die die Barriereschicht im Stratum Corneum bilden. Durch diese "Aufweichung" gelingt es den kleinen flexiblen Liposomen leichter in die tieferen Hautschichten einzudringen ("Ethosomal Carriers"; E. Touitou und andere; Drug Dev. Res, 50 (2000)406-415).

Ein anderes "altes" Trägersystem sind die Niosomen, die mindestens aus einem nicht ionischen Detergens bestehen. Meistens enthalten sie noch einen Membranstabilisator (Cholesterol) und einen Ladungsträger (Dicetylphosphate), die die Aggregation der Vesikel verhindern sollen. Der am häufigsten eingesetzte Membranbildner ist Sorbitan Monooleate (Span 80), aber folgende Detergentien sind ebenfalls beschrieben: Sucrosemonostearate, Sucrosedistearate, POE(5)Sorbitantrioleate und Dioleylgluconate. Exemplarisch werden hier einige Patente zu Niosomen aufgeführt: die US Patentanmeldung US 4536324 (A) beschreibt Niosomen aus PEG-10 Castoröl mit Sorbitantrioleate; die US Patentanmeldung US 4830857 (A) beschreibt die Herstellung eines nicht ionischen Detergens und Niosomen aus diesem Stoff mit Cholesterol und Dicetylphosphate; die US Patentanmeldung US 5405615 (A) beschreibt Niosomen aus Sucrosedistearate und einem Fettalkohol-Derivat; die DE-Patentanmeldung 69507488 beschreibt Niosomen aus PEG-2- bis PEG-6-Monohexadecylether, Cholesterol und Dicetylphosphate; die US Patentanmeldung US 5720948 (A) beschreibt Niosomen aus Glyceryldilaurate, Cholesterol und PEG-10-Stearylester; die US Patentanmeldung 20070269379 (A) beschreibt Niosomen aus N-Laurylsarcosine und Sorbitanmonolaurate (Span 20) und WO 2007/123993 beschreibt Niosomen aus Sorbitanmonostearate (Span 60), PEO-Sorbitanmonostearate (Tween 61), Cholesterol und Dicetylphosphate.

Besonders hervorzuheben ist die US Patentanmeldung US 5830499 (A), die Niosomen beschreibt, welche Wirkstoffe besonders tief in die Haut transportieren können. Die einschaligen Vesikel bestehen aus flexiblen Membranen, die aus einem membranbildenden, nicht ionischen Detergens und einem zweiten nicht ionischen, hydrophilen Emulgator gebildet werden. Hier wurde die Penetration der Vesikel selbst in den tieferen Hautschichten nachgewiesen.

Ein weiterer Wirkstoffträger für den dermalen Einsatz wird beschrieben in der EP 1060732 A1; es sind multilamellare Niosomen bestehend aus einem zweikettigen Lipid, optional aus einem einkettigen Lipid und einem Sterol. Die hier bevorzugte Zusammensetzung liegt bei 30 - 40% Glyceryldistearate, 29-37% POE-10 Stearylether und 11 -14% Cholesterol. Hier wird die Erhöhung der Wirkstoffkonzentration in der Epidermis mittels dieses Trägersystems aufgeführt.

Ein in der Kosmetik eingesetztes multilamellares Trägersystem mit optimierter Wirkstofffreigabe (Time Release Effekt) stellt das Spherulites® Trägersystem (Firma Impag) dar. Dieses Trägersystem wird in FR 2771635 (A1) beschrieben und besteht zu 30 - 50% aus Tensiden pflanzlichen Ursprungs (Zuckerester wie Sucrosepalmitat oder Sucrosedistearate).

WO 00/12108 A1 offenbart Emulsionen, welche Imwitor 375, Lipide und Wirkstoffe enthalten können. Derartige Emulsionen werden auch in "Product Information Imwitor 375", Sasol Germany GmbH, April 2000, offenbart.

US 6,033,710 A offenbart Vesikel, welche Diacetyl Tartaric Acid Monostearate Glyceride und Lipide enthalten.

Bisher noch nicht beschrieben sind Phospholipid-freie Vesikel, bestehend aus einem besonders hautfreundlichen, pflanzlichen und lebensmittelrechtlich zugelassenen membranbildenden anionischen Emulgator in Kombination mit einem weiteren einkettigen Lipid zur Stabilisierung der Vesikel.

Es ergab sich somit die Aufgabe, eine geeignete Lipidzusammensetzung zu finden, die ein stabiles aber auch flexibles Trägersystem ermöglicht, das die Fähigkeit aufweist, Wirkstoffe (lipophil, amphiphil oder hydrophil) in die tieferen Hautschichten zu transportieren.

Gelöst wird die Aufgabe durch die erfindungsgemäßen Vesikel, die die folgenden Bestandteile enthalten:
- einen wässerigen Kern, der mindestens einen kosmetischen und / oder mindestens einen pharmazeutischen Wirkstoff aufweist und
- eine den Kern umgebende kontinuierliche Membran, gebildet aus mindestens einem Emulgator, ausgewählt aus der Gruppe der Lebensmittelzusatzstoffe E 472 a bis 472 f, und mindestens einem membranstabilisierenden einkettigen Lipid und einem oder mehreren lipophilen kosmetischen und/oder pharmazeutischen Wirkstoffen, wobei es sich bei dem Membran bildenden Emulgator um Glyceryl Citrate / Lactate / Linoleate / Oleate (Imwitor 375) handelt.

Die Aufgabe wird ferner gelöst durch eine die Vesikel enthaltende Zubereitung.

Die erfindungsgemäßen einschaligen Vesikel weisen eine negative Ladung (-30 bis -60 mV) auf und haben vorzugsweise eine Größe von 80 - 400 nm. Sie liegen dispergiert in einer wässerigen Lösung vor.

Die vorliegende Erfindung weist eine Verkapselungseffizienz auf, die sich von der normalen liposomalen Verkapselung dadurch auszeichnet, dass Wirkstoffe wie Polyphenole (Phenolsäuren und Flavonoide) in höherer Konzentration stabil im Vesikel eingeschlossen werden können.

Weitere Vorteile der erfindungsgemäßen Zubereitung sind die hohe Stabilität der Komposition auch in Emulgator-haltigen kosmetischen und pharmazeutischen Formulierungen, ein gutes Hautgefühl und schnelles Eindringen in die Haut.

Die erfindungsgemäß verwendbaren membranbildenden Emulgatoren sind für den menschlichen Verzehr geeignet. Anionische Emulgatoren ausgewählt aus der Gruppe der Lebensmittelzusatzstoffe E 472 a bis 472 f, besonders die Ester von Diacylglyceriden, werden in der vorliegenden Erfindung verwendet. Ein erfindungsgemäß verwendeter Emulgator ist das Glyceryl Citrate/Lactate/Linoleate/Oleate (Imwitor 375). Der hohe Anteil an ungesättigten und mehrfach ungesättigten Fettsäuren ermöglicht die Bildung von Vesikeln mit einer flexiblen Membran. Die Flexibilität ist eine Voraussetzung für die Penetration der Vesikel in die Haut nach topischer Applikation. Diese Emulgatoren mit langen Alkyl-ketten zeichnen sich durch eine hohe Hautverträglichkeit aus und können kosmetische und pharmazeutische Effekte erzielen (z.B.: Erhöhung der Hautglätte, Reduzierung von Metalloproteasen, Reduzierung von Hautunreinheiten).

Vesikel, die aus reinem Glyceryl Citrate/Lactate/Linoleate/Oleate in Wasser gebildet werden, haben eine sehr negativ geladene Oberfläche (-75 mV) und sind instabil in kosmetischen Formulierungen.

Neben dem membranbildenden Emulgator wird ein lipophiler einkettiger Stabilisator eingesetzt, der als hydrophoben Teil langkettige Fettsäuren (bevorzugt Öl- oder Eicosapentaensäure) enthält. Dieser Stabilisator ist nötig, um höhere Einschlüsse von Wirkstoffen (z.B. Polyphenolen) zu erzielen, die Penetrationseigenschaften der Vesikel zu verbessern und die Vesikelstabilität in kosmetischen und pharmazeutischen Formulierungen zu erhöhen.

Zur Membranstabilisierung kann ein weiterer lebensmittelrechtlich zugelassener Emulgator eingesetzt werden wie: Zuckerester von Speisefettsäuren (E 473); Polyglycerinester von Speisefettsäuren (E 475) und Propylenglycolester von Speisefettsäuren (E 477) oder ein Lösungsvermittler wie die Ester einer langkettigen Fettsäure (z.B. Ölsäure oder Eicosapentaensäure). Diese stabilisierenden Zusatzstoffe mit langer Alkylkette zeichnen sich durch eine hohe Hautverträglichkeit aus und können kosmetische und pharmazeutische Effekte erzielen (z.B: Erhöhung der Hautglätte, Reduzierung von Metalloproteasen, Reduzierung von Hautunreinheiten, Erhöhung der Hautfeuchtigkeit, entzündungshemmende Eigenschaften).

Zu den membranstabilisierenden Emulgatoren gehören u.a. Sucroseoleate (Surfhope SE von Mitsubishi-Kagaku Foods Corp.), Trehalose Isostearate (Nomcort TQ 5 von Nisshin Oilliogroup), Glyceryl Monooleate (Cithrol von GMO von Croda), Di-Glyceryl Oleate (Nikkol DGMO-CV von Nikko Chemicals) und Polyglyceryl-4-Oleate (Hydriol PGMO von Hydrior).

Zu den membranstabilisierenden Lösungsmitteln (Ersatz für Öle) gehören u.a. Ethyloleate (Crodamol EO von Croda) und der Ethylester der Eicosapentaensäure (EPA 95 EE von Equateq).

Besonders bevorzugt werden Ethyloleate (EO) und / oder Ethylester der Eicosapentaensäure (EE) zur Stabilisierung der Vesikelmembran eingesetzt.

Die Menge an membranbildendem Emulgator beträgt zwischen 3 und 12 Gew.-%, besonders bevorzugt zwischen 6 und 8 Gew.-%, und die Menge des membranstabilisierenden einkettigen Lipids (OE und / oder EE) zwischen 2 und 6 Gew.-%, bezogen auf die Vesikel enthaltende Zubereitung. Beide Komponenten bilden die Schale der Vesikel.

Die erfindungsgemäßen Vesikel können zusätzlich noch einen oder mehrere lipophile Wirkstoffe in der Vesikelmembran enthalten. Als solche Substanzen sind alle Stoffe anzusehen, die sich in einem lipophilen Medium bei Raumtemperatur oder unter Wärme lösen lassen und kosmetisch und/oder pharmazeutisch Effekte in oder auf der Haut ausüben.

Ein Auszug der kosmetisch und / oder dermatologisch wirksamen lipophilen Substanzen, die eingesetzt werden können - bevorzugt pflanzlichen Ursprungs - sind: Anti-Cellulite wirksame Substanzen (z.B.: CLA); Anti-Elastase und Anti-Collagenase wirksame Stoffe (z.B.: ungesättigte Fettsäuren wie Ölsäure oder EPA); anti-inflammatorisch wirksame Stoffe (z.B.: EPA = Eicosapentaensäure); Antioxidantien (z.B.: Vitamin C-Palmitate und Tocopherol; α-Liponsäure), Ceramide (z.B.: verschiedene Ceramide der Firma Cosmoferm); hautberuhigende und hautglättende Wirkstoffe (z.B.: Bisabolol); Moisturiser (z.B.: Glycerol Monoisostearate, Sucrose Polysoyate); Phytosterole (wie *β*-Sitosterol aus Maisfaseröl); Radikalfänger (z.B.: Ubichinol-Derivate wie Coenzym Q10); Saponine (z.B.: vom Ginseng, Süßholzwurzel und Hydroxydecansäure); Stoffe, die die Durchblutung und damit die Versorgung der Haut fördern (z.B.: lipophile Nikotinsäureester); Terpene (Ursolsäure, Rosmarinsäure, Betulinsäure, Boswelliasäure und Bryonolsäure; kosmetisch und dermatologisch relevante Terpene sind aufgeführt in der Pharmazeutischen Zeitung Heft 22; 2006 von Sebastian Jäger u.a.); Vitamine (Retinol und Derivate, Vitamin E und Derivate wie Tocotrienole oder Carotine und Carotinoide wie Lycopene, Lutein oder Fucoxanthin, Vitamin D und Derivate) und lipophile Peptide als Anti-Ageing Produkt (z.B. Ester von 2-10 Aminosäuren, die an Palmitinsäure gebunden sind).

Alle diese Wirkstoffe sollen die Hautalterung und / oder das Photoageing (durch UV- bzw. Umweltbelastung) verhindern bzw. inhibieren; die Synthese von dermalen und epidermalen Makromolekülen stimulieren bzw. deren Degradation verhindern; die Proliferation von Fibroblasten und Keratinozyten anregen und somit den Schutz und die Erhaltung einer gesunden Haut bewirken.

Pharmazeutisch eingesetzte erfindungsgemäße Vesikel enthalten eine sichere und wirksame Menge eines pharmazeutisch wirksamen Stoffes, welcher unter den Arzneimitteln gegen Akne (z.B. Isotretinoin und Isolutrol), nichtsteroidalen entzündungshemmenden Mitteln (z.B. Diclofenac), Mitteln zur Heilung von Wunden, Mitteln zur Behandlung von Hauterkrankungen / Ekzeme (z.B. Corticosteroide), Lokalanästhetika (z.B. Lidocain), Mitteln gegen Pilzinfektionen (z.B. Terbinafin), antimikrobiellen Arzneimitteln, Mitteln gegen Psoriasis (z.B. Calcipotriol), Mitteln zur Stimulierung des Haarwuchses (z.B. Minoxidil), Mitteln gegen chronische venöse Insuffizienz, Mitteln gegen Herpesinfektionen (z.B. Aciclovir), Antihistaminika (z.B. Dimetinden) und Mitteln gegen Hautkrebs (z.B. Diclofenac und Psoralene) zu finden ist.

Alle diese relevanten pharmazeutischen Wirkstoffe sollen mittels des Trägersystems effektiv in die tieferen Hautschichten transportiert werden, was zu einer höheren Bioverfügbarkeit am Wirkort führt. Damit kann zum Teil die Wirkstoffkonzentration gesenkt werden und eventuelle Nebenwirkungen vermieden werden. Ferner kann die Verkapselung der Wirkstoffe diese stabilisieren und auch Nebenwirkungen wie Hautirritationen (z.B. bei Vitamin A-Derivaten) verhindern.

In einer erfindungsgemäß bevorzugten Ausführungsform liegt das Gewichtsverhältnis von lipophilen Bestandteilen in der Membran beim membranbildenden Emulgator zu Ethyloleate (Eicosapentaensäure Ethylester) und / oder Wirkstoff im Bereich von 1:1 bis 4: 1.

Die erfindungsgemäßen Vesikel enthalten mindestens einen kosmetischen und / oder pharmazeutischen Wirkstoff, der im wässerigen Innenkern vorliegen oder in der Membran gebunden sein kann. Die Vorbereitung der erfindungsgemäßen Vesikel erfolgt durch Dispergieren einer der die Lipide enthaltenden Lösung in einer wässerigen Lösung (Trägervehikel), die mindestens einen hydrophilen Wirkstoff enthalten kann. Die Vesikel liegen dann im wässerigen Trägervehikel vor und der hydrophile Wirkstoff ist sowohl in den Vesikeln selbst als auch im wässerigen Trägervehikel vorhanden. Das Vorhandensein des Wirkstoffes in der die Vesikel umgebenden wässerigen Phase ermöglicht eine Konzentrierung der dort befindlichen Wirkstoffe an der Vesikeloberfläche und damit einen höheren Wirkstofftransport in die Haut.

Kosmetische und pharmazeutische Wirkstoffe können u.a. aus folgenden Stoffgruppen ausgewählt sein:

### Aminosäuren, Peptide, Proteine und Enzyme:

Die erfindungsgemäßen Zusammensetzungen können Monomere, Oligomere und Polymere von Aminosäuren sowie Ester und/oder physiologisch verträgliche Metallsalze dieser Substanzen enthalten.

Die Monomere der Aminosäuren können ausgewählt sein aus Alanin, Arginin, Asparagin, Asparaginsäure, Canavanin, Citrullin, Cystein, Glutamin, Glycin, Histidin, Hydroxylysin, Hydroxyprolin, Leucin, Lysin, Methionin, Prolin, Serin, Threonin, Tyrosin und Valin.

Die Oligomeren der Aminosäuren sind bevorzugt ausgewählt aus Di-, Tri-, Tetra-, Penta-, Hexa- oder Pentadecapeptiden, die acyliert und/oder verestert sein können. Zahlreiche dieser Aminosäureoligomere stimulieren die Collagensynthese und werden bevorzugt als Wirkstoffe gegen die Hautalterung verwendet.

Die Polymeren der Aminosäuren sind ausgewählt aus pflanzlichen und tierischen Proteinhydrolysaten und/oder Proteinen. Tierische Proteinhydrolysate sind z.B. Elastin-, Collagen-, Keratin-, Seiden- und Milcheiweiß-Proteinhydrolysate, die auch in Form von Salzen vorliegen können. Pflanzliche Proteinhydrolysate können sein: z.B. Soja-, Weizen-, Mandel-, Erbsen-, Kartoffel- und Reisproteinhydrolysate.

In einer weiteren Ausführungsform können Enzyme verkapselt sein, ausgewählt u.a. aus DNA-Reparaturenzymen (z.B. die Photolyase); anti-oxidativ wirkenden Enzymen (u.a. Superoxydismutase), Proteasen (u.a. Trypsin) und Lipasen.

### Wässerige und/oder alkoholische Extrakte aus Pflanzen, Pilzen oder Algen:

Der Pflanzenextrakt kann beispielsweise durch Extraktion der gesamten Pflanze, aber auch ausschließlich durch Extraktion aus Blüten und/oder Blättern und/oder Samen und/oder anderen Pflanzenteilen, hergestellt werden. Die verwendeten Algenextrakte stammen u.a. aus Grünalgen, Braunalgen, Rotalgen oder Blaualgen (Cyanobakterien). Die zur Extraktion eingesetzten Algen und Pilze (speziell Hefen) können sowohl natürlichen Ursprungs sein als auch durch biotechnologische Prozesse gewonnen werden und gewünschtenfalls gegenüber der natürlichen Form verändert sein. Als Extraktionsmittel zur Herstellung der Pflanzenextrakte können beispielsweise Wasser, Alkohole sowie deren Mischungen verwendet werden. Die Wasserdampfdestillation fällt erfindungsgemäß unter die bevorzugten Extraktionsverfahren.

### Oligonukleotide:

Die erfindungsgemäßen Zusammensetzungen können Oligonukleotide enthalten. Mononucleotide sind u.a. Adenosinphosphate, Cytidinphosphate, Guanosinphosphate, Uridinphosphate und Thymidinphosphate, insbesondere CMP (Cytidin-5'-monophosphat), UDP (Uridin-5'-diphosphat), ATP (Adenosin-5'-triphosphat) und GTP (Guanosin-5'-triphosphat).
Ein erfindungsgemäß besonders bevorzugtes Oligonucleotid ist das Thymidin-Dinucleotid (pTT), das DNA-reparierende Eigenschaften aufweist als auch bräunend wirkt.

### Zuckerbestandteile:

Weiterhin können die erfindungsgemäßen Zusammensetzungen ein Mono-, Oligo- oder Polysaccharid oder deren Derivate enthalten.

Geeignete Monosaccharide sind z.B. Glucose, Fructose, Galactose, Arabinose, Ribose, Xylose, Lyxose, Allose, Altrose, Mannose, Gulose, Idose und Talose, die Desoxyzucker Fucose und Rhamnose sowie Aminozucker wie z. B. Glucosamin oder Galactosamin.

Erfindungsgemäß geeignete Polysaccharide sind aus mehr als zehn Monosaccharideinheiten zusammengesetzt. Besonders bevorzugt eingesetzt werden können u.a. die Mucopolysaccharide (Hyaluronsäure und ihre Derivate, Chondroitin und seine Derivate, Heparin und seine Derivate, Fucoidan und seine Derivate, Keratan und seine Derivate) und die β-Glucane.

Ein besonders bevorzugter Komplex verschiedener Zuckervarianten ist das Tonsolin (von Gova Group), das Mannose, Glucose, *β*-Glucan und Hyaluronsäure enthält und eine schnelle und sichere Bräunung der Haut erzielt.

### Hydroxy- und Ketocarbonsäuren und deren Salze:

Weitere bevorzugte hydrophile Wirkstoffe sind die α-Hydroxycarbonsäure, α-Ketocarbonsäure oder *β*-Hydroxycarbonsäure oder deren Ester-, Lacton- oder Salzform. Dazu gehören u.a. Glycolsäure, Milchsäure, Weinsäure, Citronensäure, 2-Hydroxyhexadecansäure, Mandelsäure, Äpfelsäure, Glucarsäure, Galactarsäure, Mannarsäure, Gluconsäure, Brenztraubensäure, Salicylsäure, Glucuronsäure und Galacturonsäure.

### Vitamine:

In einer weiteren Ausführungsform können die erfindungsgemäßen Zusammensetzungen ein wasserlösliches Vitamin enthalten aus den Vitamingruppen B und C und den Estern der vorgenannten Substanzen.

Zur Vitamin B-Gruppe oder zu dem Vitamin B-Komplex gehören unter anderem Vitamin B₁ (Thiamin), Thiaminhydrochlorid, Vitamin B₂ (Riboflavin), Riboflavin oder seine Derivate, Vitamin B₃ (Nicotinsäure und Nicotinsäureamid), Vitamin B₆- Gruppe (Pyridoxin, Pyridoxamin und Pyridoxal) und Vitamin B₇ (Biotin).

Vitamin C (Ascorbinsäure) wird bevorzugt als folgende stabile Derivate eingesetzt: Ascorbylacetat, Mg-Ascorbylphosphat, Na-Ascorbylphosphat, Natrium- und Magnesiumascorbat, Dinatrium-Ascorbylphosphat und - sulfat, Kaliumascorbyltocopherylphosphat und Ascorbylglucosid.

### Polyphenole:

Polyphenole sind aromatische Verbindungen, die zwei oder mehr direkt an den aromatischen Ring gebundene Hydroxylgruppen enthalten und zu den sekundären Pflanzenstoffen gerechnet werden. Natürliche Polyphenole kommen in Pflanzen als bioaktive Substanzen wie Farbstoffe (Flavonoide, Anthocyane), Geschmacksstoffe und Tannine vor. Zu den Polyphenolen gehören die Phenolsäuren und die Flavonoide, die aufgrund ihrer kosmetischen und/oder pharmazeutischen Wirkung (u.a. antioxidative, entzündungshemmende, antibakterielle und antivirale Eigenschaften) in der Vesikel verkapselt werden können.

Zu den erfindungsgemäß einsetzbaren Phenolsäuren gehören u.a. Gallussäure, Vanillinsäure, Usninsäure, Ferulasäure, Ellagsäure und Kaffesäure.

Zu den erfindungsgemäß einsetzbaren Flavonoiden und/oder deren glycosidische Derivate gehören: u.a. Flavone (Luteolin, Apigenin), Flavonole (Baicalin, Quercetin, Rutin, Kaempferol, Myricetin), Flavanole (Catechin, Gallocatechin, Epicatechin, Epigallocatechingallat, Resveratol, Silymarin, Theaflavin), Flavanone (Aspalathin, Hesperetin, Naringenin), Flavanonole (Taxifolin), Isoflavone (Genistein, Daidzein, Licoricidin) und die Anthocyanidine oder Anthocyane (natürliche Farbstoffe).

Eine besonders bevorzugte Kombination für den Einschluss in die Vesikel ist das Univestin (von Unigen), bestehend aus vorwiegend Baicalin und Catechin, welches sich durch ein hohes anti-oxidatives Potential und gute anti-inflammatorische Eigenschaften auszeichnet.

### Bräunung:

Erfindungsgemäß können in die Vesikel folgende Wirkstoffe zur Induzierung der Pigmentierung eingesetzt werden: Erythrulose; Unipertan Veg (Gemisch aus Acetylthyrosin, Riboflavin und hydrolysiertem pflanzlichen Protein von Induchem); Tonsolin (Zuckermischung von Gova), das Dinucleotid pTT und das melaninstimulierende Peptid (α-Melanotropin).

### Hautaufheller:

In einer weiteren bevorzugten Ausführungsform kann die erfindungsgemäße Zusammensetzung ein hautaufhellenden Wirkstoff, der Ascorbinsäure oder deren Ester, enthalten (Natriumascorbylphosphat und Magnesiumascorbylphosphat) oder das Hydroxytyrosol (Cayoma Olive von Qenax).

### Antioxidantien:

Die erfindungsgemäßen Zusammensetzungen können ferner Antioxidantien enthalten, zum Beispiel Imidazole (z.B. Urocaninsäure) und deren Derivate; Peptide (u.a. D,L-Carnosin, D-Carnosin, L-Carnosin); Säuren und deren Derivate (Chlorogensäure, Huminsäure, Gallensäure, Rutinsäure und Ferulasäure); Vitamine (Vitamin C und seine Derivate wie Magnesiumascobylphosphate); Enzyme (Katalase und Superoxid-Dismutase) Zink und dessen Derivate (z.B. ZnO, ZnSO₄); Selen und deren Derivate (z.B. Selen-Methionin); Stilbene und deren Derivate (z.B. Resveratol).

Alle diese Wirkstoffe sollen die Hautalterung und / oder das Photoageing (durch UV- bzw. Umweltbelastung) verhindern bzw. inhibieren; die Synthese von dermalen und epidermalen Makromolekülen stimulieren bzw. deren Degradation verhindern; die Proliferation von Fibroblasten und Keratinozyten anregen und somit den Schutz und die Erhaltung einer gesunden Haut bewirken.

In einer erfindungsgemäß bevorzugten Ausführungsform liegt das Gewichtsverhältnis der lipophilen Bestandteile der Membran bei 5-15 Gew.-% (Imwitor: Stabilisator: Wirkstoff), besonders bevorzugt zwischen 8 und 10 Gew.-%, und die des eingesetzten hydrophilen Wirkstoffes zwischen 0,1 bis 20 Gew.-%, bezogen auf die Gesamtzubereitung. Die Einsatzkonzentration des hydrophilen Wirkstoffes kann variieren und ist stark abhängig vom jeweiligen Lösungsverhalten des Stoffes.

Die erfindungsgemäßen Zubereitungen können weiterhin wenigstens einen Stabilisator, der vorzugsweise ein wasserlöslicher Alkohol ist, enthalten. Der Stabilisator / Solvent wird vorzugsweise in einer Konzentration von 10-20 Gew.₋%, bezogen auf die gesamte Zubereitung, eingesetzt. Geeignet sind je nach Darreichungsform einwertige Alkohole wie z.B. Ethanol, Propanol, Isopropanol oder natürlicher Phenylethylalkohol. Weiterhin geeignet sind wasserlösliche Polyole. Hierzu zählen wasserlösliche Diole, Triole und höherwertige Alkohole. Unter den Diolen eignen sich C₂- bis C₁₀- Diole, insbesondere 1,2-Propylenglycol, 1,2-Butylenglycol, 1,3-Butylenglycol und 1,4-Butylenglycol, 1,2-Pentadiol, 1,6-Hexandiol und 1,2 Octandiol. Weiterhin bevorzugt geeignet sind Glycerin und insbesondere Diglycerin und Triglycerin, 1,2,6-Hexantriol sowie die Dipropylenglycole. Für die Herstellung der erfindungsgemäßen Vesikel werden bevorzugt Ethanol, Phenylethylalkohol oder 1,2 Pentandiol (Hydrolite 5 von Symrise) eingesetzt, in Konzentrationen von 10 bis 30 Gew.-%, besonders bevorzugt von 15-20 Gew.-%, bezogen auf die gesamte Formulierung, so dass somit auf eine andere Konservierung (klassische Konservierungsmittel) verzichtet werden kann. Die erfindungsgemäße Zubereitung kann deshalb als konservierungsmittelfrei deklariert werden.

Die Vesikel enthaltende wässerige Zubereitung kann selbst als Kosmetikum / Pharmazeutikum eingesetzt werden, je nach enthaltenem Wirkstoff. Darüber hinaus lässt die Vesikel enthaltende wässerige Zubereitung sich in die Wasserphase einer weiteren Zubereitung wie eine Emulsion, Lotion, Creme oder unterschiedliche Formen von Gelen einarbeiten oder kann selbst verdünnt mit einem zugesetzten Verdicker als Sprühformulierung angewendet werden.

Die hier beschriebene Erfindung beinhaltet ebenfalls den Gebrauch der Vesikel in kosmetisch, dermatologisch und pharmazeutisch interessanten wässerigen Formulierungen (Sprühformulierung, unterschiedlichste Gele, Lotionen und Cremes) sowie deren Anwendung zur Behandlung, zum Schutz und Pflege der Haut, Haare, Nägel und Lippen.

Im Folgenden soll das Verfahren zur Herstellung einer erfindungsgemäßen Zubereitung anhand von einigen Ausführungsbeispielen und Abbildungen, die nicht als einschränkend zu verstehen sind, demonstriert werden.
Fig. 1 zeigt Grüntee verkapselt in Liposomen bzw. in Vesikel EO eine Woche nachdem diese hergestellt wurden.
Fig. 2 zeigt die UV-photometrischen Spektren der Ferulasäure, sowohl allein als auch wenn sie in den Imwitorvesikeln eingeschlossen ist.
Fig. 3 zeigt das Skin antioxidative Potential der Ferulasäure, sowohl allein als auch wenn sie in verschiedene Trägersysteme eingeschlossen ist.

### Beispiel 1

### Verkapselungen des hydrophilen Wirkstoffes "Grüntee" (KOSME Green Tea Extract)

Alle Formulierungen enthalten: 10% Lipid, 16% Äthanol, 2.5% Grüntee Extrakt und 71.5% Wasser
Zunächst wurde eine Stammlösung angesetzt aus 8 g Äthanol + 2,5 g Grüntee Extrakt + 71,5 g Wasser.

### Liposomen:

10 g Phospholipide (Phospholipon 85 G von Lipoid) wurden in 8 g Äthanol gelöst. Diese Lösung wurde unter Homogenisieren zu 82 g Grünteestammlösung gegeben. Anschließend wurde alles für 5 Minuten bei 200 bar hochdruckhomogenisiert.
Teilchengröße: 211 nm ± 0,409 (extrem inhomogene Verteilung); pH = 6.18

### Vesikel EO (Vesikel enthaltend Ethyloleate):

7 g Imwitor 375 + 3 g Crodamol EO wurden in 8 g Äthanol gelöst. Diese Lösung wurde unter Homogenisieren zu 82 g Grünteestammlösung gegeben. Anschließend wurde alles für 5 Minuten bei 200 bar hochdruckhomogenisiert.
Teilchengröße: 122 nm ± 0,091 (sehr homogene Verteilung); pH = 6.1

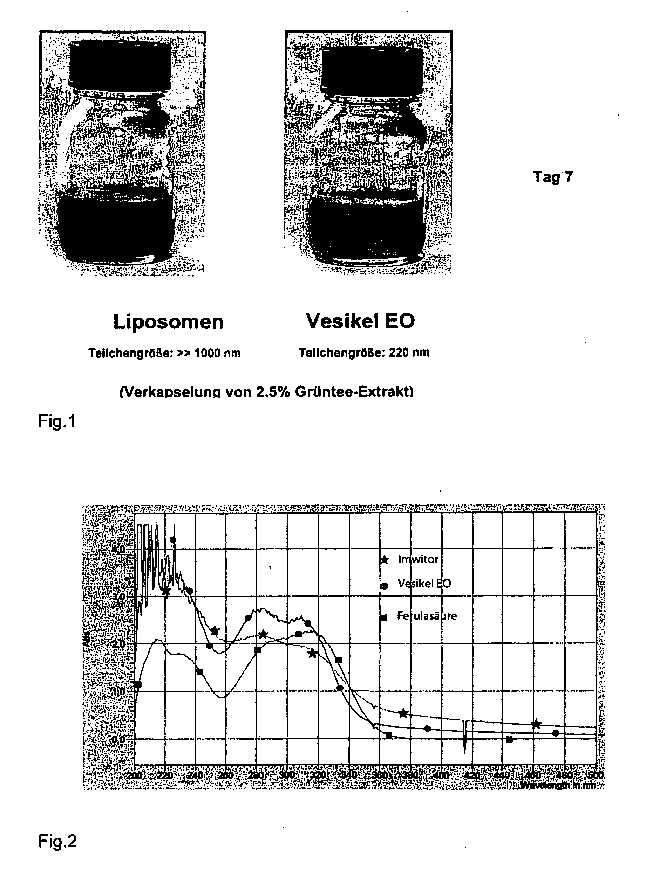

Nach einer Woche zeigte sich ein starker Bodensatz bei den Liposomen mit Grüntee während die Vesikel EO eine stabile Formulierung darstellen, wie aus Fig. 1 ersichtlich.

### Beispiel 2

### Verkapselung des amphiphilen Wirkstoffs "Ferulasäure"

### Liposomen:

1 g Ferulasäure (GfN) wurde zunächst in 16 g Äthanol gelöst und anschließend 10 g Phospholipide (Phospholipon 85 G von Lipoid). Diese Lösung wurde unter Homogenisieren zu 73 g Phosphatpuffer (pH 7.2) gegeben. Anschließend wurde alles für 5 Minuten bei 200 bar hochdruckhomogenisiert.
Teilchengröße: 85 nm ± 0,386 (sehr inhomogene Verteilung); pH = 5.7 Nach 2 Wochen Schlierenbildung!

### Reiner Imwitor:

1 g Ferulasäure (GfN) wurde zunächst in 16 g Äthanol gelöst und anschließend 10 g Imwitor 375. Diese Lösung wurde unter Homogenisieren zu 73 g Wasser gegeben. Anschließend wurde alles für 5 Minuten bei 200 bar hochdruckhomogenisiert.
Teilchengröße: 174 nm ± 0,213 (homogene Verteilung); pH = 5.3

### Vesikel EO:

1 g Ferulasäure (GfN) wurde zunächst in 16 g Äthanol gelöst und anschließend 7 g Imwitor 375 + 3 g Crodamol EO. Diese Lösung wurde unter Homogenisieren zu 73 g Wasser gegeben. Anschließend wurde alles für 5 Minuten bei 200 bar hochdruckhomogenisiert. Teilchengröße: 124 nm ± 0,068 (sehr homogene Verteilung); pH = 5.6

### Vesikel EE (Vesikel enthaltend Ethylester der Eicosapentaensäure):

1 g Ferulasäure (GfN) wurde zunächst in 16 g Äthanol gelöst und anschließend 7 g Imwitor 375 + 2 g Crodamol EO + 1 g EPA 95 EE. Diese Lösung wurde unter Homogenisieren zu 73 g Wasser gegeben. Anschließend wurde alles für 5 Minuten bei 200 bar hochdruckhomogenisiert. Teilchengröße: 124 nm ± 0,060 (sehr homogene Verteilung); pH = 5.67

Von allen diesen Formulierungen wurde das antioxidative Potential (AP) in vitro gemessen. Dabei wird die Formulierung mit einem Testradikal inkubiert (DPPH) und die Reduktion dieses Radikals über die Zeit mittels ESR (Elektronen Spin Resonanz Spektroskopie) bestimmt (AP = Anzahl freier Radikale / mg Testsubstanz * Minute).

| | | |
|---|---|---|
| 1 % Ferulasäure (EtOH/ Wasser): | AP = | 723 |
| 1 % Ferulasäure Liposomen: | AP = | 2.220 |
| 1 % Ferulasäure Imwitor: | AP = | 980 |
| 1 % Ferulasäure Vesikel EO: | AP = | 2.073 |
| 1 % Ferulasäure Vesikel EE: | AP = | 2.017 |

Hier wird ersichtlich, dass der reine Imwitorvesikel nur eine geringe aktivierende Wirkung auf die Ferulasäure ausübt (Faktor 1,36) während die anderen stabilen Membranen das AP der Ferulasäure um den Faktor 3,1 für die Liposomen und 2,8 für die Vesikel EO / EE erhöhen können. UV-photometrische Spektren zeigen eine Veränderung der Absorptionsmaxima der Ferulasäure, wenn sie in den Imwitorvesikeln eingeschlossen wird (Fig. 2).

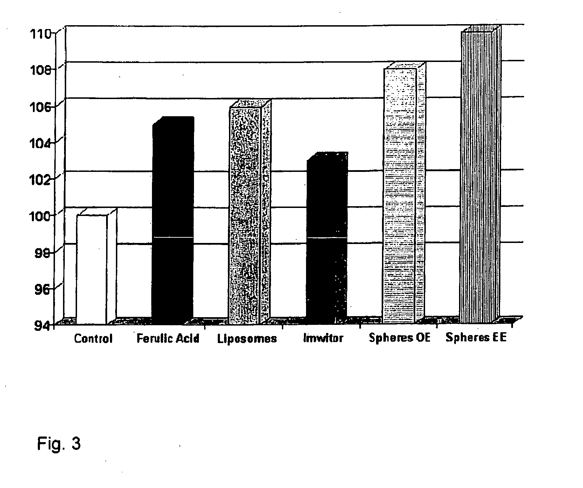

Eine Messung der Penetration von Ferulasäure mittels der verschiedenen Trägersysteme in die Haut wurde mit Hilfe der ESR-Spektroskopie bestimmt. Die Haut hat einen natürlichen anti-oxidativen Schutz, gegeben durch hauteigene enzymatische und nicht-enzymatische Wirksubstanzen. Dieses anti-oxidative Potential der Haut (Skin Antioxidative Potential, SAP) kann gemessen werden, in dem die Epidermis mit Testradikalen inkubiert wird und die Reduktion der Radikale mittels ESR verfolgt wird (entspricht 100%). Topisch applizierte Antioxidantien, die in die Haut penetrieren können, können dann das SAP erhöhen. Einzig die Vesikel EE und EO zeigen eine signifikante Erhöhung des SAP (Fig. 3).

### Beispiel 3

### Verkapselung des lipophilen Wirkstoffs "Diclofenac"

### Spheres EE:

2 g Diclofenac wurde zunächst in 16 g Äthanol gelöst und anschließend 7 g Imwitor 375 + 1 g EPA 95 EE. Diese Lösung wurde unter Homogenisieren zu 73 g Wasser gegeben. Anschließend wurde alles für 5 Minuten bei 200 bar hochdruckhomogenisiert.
Teilchengröße: 135 nm ± 0,096 (sehr homogene Verteilung).

### Beispiel 4

### Stabilität der Vesikel in einer kosmetischen Formulierung

Hierbei wird die Integrität der Vesikelmembran in kosmetischen Fertigformulierungen mittels ESR-Spektroskopie bestimmt. Dazu wird in die Vesikelmembran ein ESR-aktiver Label eingebaut, der unterschiedliche ESR-Signale aufweist bei Verkapselung in der Membran und frei vorliegend in der Formulierung.

| | Stabilität in % | | |
|---|---|---|---|
| | t = 0 | t = 24 h | t = 48h |
| Liposomen | 95 | 90 | 90 |
| Imwitor-Vesikel | 90 | 60 | 40 |
| Vesikel EO | 90 | 80 | 80 |

Formulierung: Water; Hydrogenated Jojoba Oil; Steareth-2; PPG-15 Stearyl Ether; Glycerin; Canola Oil; Steareth-21; Shea Butter; Dicaprylyl Ether; Cyclomethicone; Xanthan Gum.

## Patentansprüche

1. Vesikel, enthaltend mindestens die folgenden Bestandteile:
- einen wässerigen Kern, der mindestens einen kosmetischen und/oder mindestens einen pharmazeutischen Wirkstoff aufweist, und
- eine den Kern umgebende kontinuierliche Membran, gebildet aus mindestens einem Emulgator, ausgewählt aus der Gruppe der Lebensmittelzusatzstoffe E 472 a bis f, und mindestens einem membranstabilisierenden einkettigen Lipid und einem oder mehreren lipophilen kosmetischen und/oder pharmazeutischen Wirkstoffen, wobei es sich bei dem membranbildenden Emulgator um Glyceryl Citrate/ Lactate/ Linoleate/ Oleate (Imwitor 375) handelt.

2. Vesikel nach Anspruch 1,
**dadurch gekennzeichnet,**
**dass** sie einschalig sind, ein negatives Oberflächenpotential aufweisen und eine Größe von 80 bis 400 nm besitzen.

3. Vesikel nach einem der Ansprüche 1 bis 2,
**dadurch gekennzeichnet,**
**dass** es sich bei dem membranstabilisierenden einkettigen Lipid bevorzugt um den Ethylester der Ölsäure und/oder den Ethylester der Eicosapentaensäure handelt.

4. Vesikel nach Anspruch 3,
**dadurch gekennzeichnet,**
**dass** das Gewichtsverhältnis von Emulgator zu Membranstabilisator und/oder lipophilem Wirkstoff bei 1:1 bis 4:1 liegt.

5. Zubereitung enthaltend Vesikel nach einem der Ansprüche 1 bis 4,
**dadurch gekennzeichnet,**
**dass** das Trägervehikel der Zubereitung wässerig ist, wobei das wässerige Trägervehikel und der wässerige Vesikelkern eine gleiche Zusammensetzung aufweisen.

6. Zubereitung nach Anspruch 5,
**dadurch gekennzeichnet,**
**dass** sie weiterhin mindestens einen hydrophilen Stabilisator enthält.

7. Zubereitung nach einem der Ansprüche 5 bis 6 zur therapeutischen Behandlung des menschlichen oder tierischen Körpers.

8. Verwendung der Zubereitung nach einem der Ansprüche 5 bis 7 für einen kosmetischen und/oder nicht-therapeutischen, dermatologischen Zweck.

## Claims

1. Vesicles , comprising at least the following components:
- an aqueous core, which comprises at least one cosmetic and/or at least one pharmaceutical active substance, and
- a continuous membrane which surrounds the core and which is formed by at least one emulsifier, selected from the group of the food additives E 472 a to f, and at least one membrane-stabilising single-chain lipid and one or more lipophilic cosmetic and/or pharmaceutical active substance(s),
wherein the membrane-forming emulsifier is glyceryl citrate/lactate/linoleate/oleate (Imwitor 375).

2. Vesicles according to claim 1,
**characterised in that**
the vesicles are unilamellar, exhibiting a negative surface potential and a vesicle size from 80 nm to 400 nm.

3. Vesicles according to claims 1 to 2,
**characterised in that**
the membrane-stabilising single-chain lipid is preferably the ethyl ester of oleic acid and/or the ethyl ester of eicosapentaenoic acid

4. Vesicles according to claim 3,
**characterised in that**
the ratio in weight of the emulsifier to the membrane stabiliser and/or the lipophilic active substance is 1:1 to 4:1.

5. Preparation comprising the vesicles according to any of the claims 1 to 4,
**characterised in that**
the dispersion vehicle of the preparation is aqueous, wherein the aqueous dispersion vehicle and the aqueous core of the vesicles exhibit the same composition.

6. Preparation according to claim 5,
**characterised in that**
the preparation further comprises at least one hydrophilic stabiliser.

7. Preparation according to claims 5 to 6 for the therapeutic treatment of human or animal bodies.

8. Application of the preparation according to any of the claims 5 to 7 for a cosmetic and/or non-therapeutic dermatological purpose.

## Revendications

1. Vésicules, comprenant au moins les composants suivants :
- un coeur aqueux qui comporte au moins une substance active cosmétique et/ou au moins une substance active pharmaceutique, et
- une membrane continue entourant le coeur, formée d'au moins un émulsifiant choisi parmi le groupe des additifs alimentaires E 472 a à f, et d'au moins un lipide à chaîne unique stabilisateur de la membrane et d'une ou plusieurs substance(s) active(s) lipophile(s) cosmétique(s) et/ou pharmaceutique(s), l'émulsifiant formant membrane étant le glyceryl citrate/lactate/linoléate/oléate (Imwitor 375).

2. Vésicules selon la revendication 1,
**caractérisées en ce qu'**
elles sont unilamellaires, ont un potentiel de surface négatif et ont une taille de 80 à 400 nm.

3. Vésicules selon l'une quelconque des revendications 1 à 2,
**caractérisées en ce que**,
de préférence, le lipide à chaîne unique stabilisateur de la membrane est l'ester éthylique de l'acide oléique et/ou l'ester éthylique de l'acide eicosapentaénoïque.

4. Vésicules selon la revendication 3,
**caractérisées en ce que**
le rapport pondéral de l'émulsifiant au stabilisateur de membrane et/ou à la substance active lipophile est de 1:1 à 4:1.

5. Préparation comprenant des vésicules selon l'une quelconque des revendications 1 à 4,
**caractérisée en ce que**
le véhicule porteur de la préparation est aqueux, le véhicule porteur aqueux et le coeur de vésicule aqueux ayant une même composition.

6. Préparation selon la revendication 5,
**caractérisée en ce qu'**
elle comprend en outre au moins un stabilisateur hydrophile.

7. Préparation selon l'une quelconque des revendications 5 à 6 pour le traitement thérapeutique du corps humain ou animal.

8. Usage de la préparation selon l'une quelconque des revendications 5 à 7 pour un but dermatologique cosmétique et/ou non thérapeutique.
